# EUROPEAN PATENT SPECIFICATION

(11) **EP 3 793 694 B1**
(45) Date of publication and mention of the grant of the patent: **11.05.2022**
(21) Application number: 19722643.4
(22) Date of filing: 13.05.2019
(51) Int. Cl.: A61Q 17/04, A61Q 19/00, A61K 8/06, A61K 8/42, A61K 8/49, A61K 8/895

(54) **TOPICAL COMPOSITION**
TOPISCHE ZUSAMMENSETZUNG
COMPOSITION TOPIQUE

(30) Priority: 18.05.2018 EP 18173223
(43) Date of publication of application: 24.03.2021
(73) Proprietor: DSM IP Assets B.V., 6411 TE Heerlen (NL)
(72) Inventor: MENDROK-EDINGER, Christine, 4303 Kaiseraugst (CH); RADOMSKY, Karina, 4303 Kaiseraugst (CH)
(74) Representative: Berg, Katja
(86) International application number: PCT/EP2019/062203
(87) International publication number: WO 2019/219604

(56) References cited:
- EP-A1- 2 921 157
- EP-A1- 3 228 299
- EP-A1- 3 260 113
- EP-A1- 3 269 425
- EP-A1- 3 311 791
- EP-A2- 2 178 493
- EP-A2- 2 185 126
- EP-B1- 2 178 493
- EP-B1- 2 185 126
- WO-A2-01/45640
- WO-A2-2012/168286
- DE-A1-102008 028 664

## Description

The present invention relates to topical compositions comprising D-panthenol, bis-ethylhexyloxyphenol methoxyphenyl triazine (BEMT) and polysilicone-15.

Sun care products have evolved considerably over the years. Earlier formulations were intended to protect the user from UV-B radiation as was once thought that UV-B rays were the most important contributors to wrinkling, skin disease, and skin cancer. However, more recent studies have shown that UV-A radiation is equally or even more important in the development of solar damage and skin diseases, such as lupus erythematosus and melanoma and non-melanoma skin cancer. Thus, today's focus is towards eliminating as much of UVA (320-400 nm) and / or UVB (280-320 nm) light as possible. Consequently, there's a constantly increasing need for sun care products exhibiting high SPF's (Sun Protection Factor) and high UVA protection while being photostable.

In this respect sunscreens contain different UVB filter (such as Polysilicone-15) but also UVA filter (such as BEMT, butyl methoxydibenzoylmethane or and diethylamino hydroxybenzoyl hexyl benzoate). UVA filters are however known to stain fabrics. This is a general issue as consumers suffer from yellow stains on their T-shirts after using sunscreens. To prevent this, consumers tend to use less sunscreen but at the same time they are less protected. The adverse effects of UV radiation on skin are well known. It is therefore important for the industry to provide solutions for such kind of issues to guarantee, that consumers apply sufficient sunscreen for appropriate protection.

The problem of cloth staining of a sun care product comprising at least one UV-filter substance such as a triazine derivative, in particular BEMT is known (EP3311791 A1; EP3228299 A1; EP3260113 A1; EP3269425 A1; WO2012/168286 A2). However, none of the documents points to a solution as provided herein.

It was therefore the object of the present invention to remedy the disadvantages of the prior art and to develop topical compositions, in particular sun care products with UVA and UVB protection containing at least one UVA filter such as in particular BEMT and polysilicone-15, which facilitate (i.e. improve respectively ease) the removal of such UVA filters out of textiles contaminated therewith.

Surprisingly, it has been found that the addition of D-panthenol to sunscreens comprising BEMT in combination with polysilicone-15 significantly reduces the textile staining of said compositions after washing.

Thus, the invention relates in one aspect to topical compositions comprising BEMT, polysilicone-15 and D-panthenol, wherein the weight ratio (w/w) of D-panthenol to BEMT is at least 1.25, even more preferably at least 1.5, such as most preferably at least 1.75, e.g. at least 2. This means that if the topical composition contains 1 part per weight of BEMT it contains at least 1.25, such as even more preferably at least 1.5, such as most preferably at least 1.75, e.g. at least 2 parts per weight of D-panthenol.

The term "topical" is understood here to mean external application to keratinous substances, which are in particular the skin, scalp, eyelashes, eyebrows, nails, mucous membranes and hair, preferably the skin.

"BEMT" stands for bis-ethylhexyloxyphenol methoxyphenyl triazine (INCI) or Bemotrizinol (INN) (CAS Number 187393-00-6). BEMT acts as a broad-spectrum UV filter absorbing UVB as well as UVA rays. It has two absorption peaks, 310 and 340 nm. BEMT is suggested for use in sun, day care, alphabetic products such as BB cream, whitening products, color cosmetics.

In all embodiments of the present invention, BEMT is advantageously used in an amount selected in the range from 0.4 to 10 wt.-%., preferably in the range from 0.4 to 10 wt.-%, 0.4 to 9 wt.-%, 0.4 to 8 wt.-%, 0.4 to 7 wt.-%, 0.4 to 6 wt.-%, 0.4 to 5 wt.-%, 0.4 to 4 wt.-%, 0.4 to 3 wt.-%, 0.5 to 3 wt.-%, 0.8 to 9 wt.-%, 0.8 to 8 wt.-%, 0.8 to 7 wt.-%, 0.8 to 6 wt.-%, 0.8 to 5 wt.-%, 0.8 to 4 wt.-%, 0.8 to 3 wt.-%, such as for instance in the range from 1 to 5 wt.-%, from 1 to 3 wt.-%, or from 2 to 5 wt.-%, based on the total weight of the composition.

D-panthenol is also referred to as panthenol (INCI), dexpanthenol, provitamin B5, or (+)-(R)-2,4-dihydroxy-N-(3-hydroxypropyl)-3,3-dimethylbutyramid. D-Panthenol improves hydration, reduces itching and inflammation of the skin, improves skin elasticity, and accelerates epidermal wound healing. The D-panthenol is commercially available and sold for instance by DSM Nutritional Products Europe Ltd.

In all embodiments of the present invention, D-panthenol is advantageously used in an amount selected in the range from 0.45 to 20 wt.-%, preferably in the range from 0.45 to 18 wt.-%, 0.45 to 16 wt.-%, 0.45 to 15 wt.-%, 0.5 to 12.5 wt.-%, 0.5 to 10 wt.-%, 0.5 to 8 wt.-%, more preferably in the range from 0.5 to 6 wt.-%, for instance in the range from 0.5 to 5 wt.-%, based on the total weight of the composition.

Polysilicone-15 (INCI) is a polysiloxane-based UV filter with a chromophore residue of the benzalmalonate type. Polysilicone-15 (INCI) is commercially available under the tradename PARSOL^{®} SLX at DSM Nutritional Products Ltd.

In all embodiments according to the present invention, polysilicone-15 is advantageously used in an amount selected in the range from of 0.4 to 10 wt.-%., preferably in the range from 0.4 to 10 wt.-%, 0.4 to 9 wt.-%, 0.4 to 8 wt.-%, 0.4 to 7 wt.-%, 0.4 to 6 wt.-%, 0.4 to 5 wt.-%, 0.4 to 4 wt.-%, 0.4 to 3 wt.-%, 0.5 to 3 wt.-%, 0.8 to 9 wt.-%, 0.8 to 8 wt.-%, 0.8 to 7 wt.-%, 0.8 to 6 wt.-%, 0.8 to 5 wt.-%, 0.8 to 4 wt.-%, 0.8 to 3 wt.-%, for instance 1 to 3 wt.-%, based on the total weight of the composition.

The amount of polysilicone-15, in the topical compositions according to the present invention is preferably selected in the range 0.4 to 10 wt.-%, such as in the range from 0.5 to 8 wt.-%, such as most preferably in the range from 0.5 to 5 wt.-% or 0.5 to 3 wt.-%, based on the total weight of the composition. Further suitable ranges would be 0.2 to 10 wt.-%, 0.3 to 9 wt.-% or 0.4 to 8-wt.-%, based on the total weight of the composition.

In all embodiments of the present invention, preferably the topical compositions comprise BEMT, polysilicone-15 and D-panthenol, wherein the weight ratio (w/w) of polysilicone-15 to BEMT is selected in the range of 0.25 to 4 (i.e. 1 part per weight of BEMT to 0.25 to 4 parts per weight of polysilicone-15, such as for instance in a weight ratio of 1 (i.e. 1 part per weight of BEMT and 1 part per weight of polysilicone-15).

In a further advantageous embodiment, the amounts of BEMT and polysilicone-15 in the topical compositions according to the present invention are selected in the range from 0.5 to 3 wt.-% of BEMT and 1 to 2 wt.-% polysilicone-15, based on the total weight of the topical composition.

In a further embodiment of the invention, the topical compositions according to the present invention comprising BEMT, polysilicone-15 and D-panthenol are cosmetic or pharmaceutical compositions.

In a further embodiment of the invention, the topical compositions according to the present invention comprising BEMT, polysilicone-15 and D-panthenol are sun care products (i.e sunscreens).

A further embodiment of the invention relates to the use of the topical composition as described above, for reducing the textile (cloth) staining of BEMT, in particular after washing, and/ or facilitating the washing of a topical composition according to the present invention out of textiles contaminated with said composition. Said compositions preferably further comprise polysilicone-15.

In another embodiment, the present invention relates to the use of D-panthenol for reducing the textile (cloth) staining caused by UVA filters such as preferably BEMT, preferably in combination with polysilicone-15.

Furthermore, the invention relates to use of D-panthenol in topical compositions containing at least one UVA filter such as preferably BEMT to facilitate the washability of the UVA filter such as in particular BEMT out of textiles contaminated therewith. Said compositions preferably further comprise polysilicone-15.

A further embodiment of the invention relates to a method for reducing textile (cloth) staining caused by topical compositions comprising at least one UVA filter such as preferably BEMT, in particular after washing, said method comprising the incorporation of D-panthenol into said composition. Said compositions preferably further comprise polysilicone-15.

In another embodiment, the present invention relates to a method and use to facilitate the washability of topical preparations containing at least one UVA filter such as preferably BEMT out of textiles, said method encompassing the addition of D-panthenol to said composition. Said compositions preferably further comprise polysilicone-15.

Preferably in all uses and methods the weight ratio of D-panthenol to BEMT is at least 1.1. Even more preferably, the weight ratio of polysilicone-15 to BEMT in said uses or methods is at least 1.

The term UVA filter as used herein in particular refers to BEMT, butyl methoxydibenzoyl methane [PARSOL^{®} 1789, CAS: 70356-09-1] and diethylamino hydroxybenzoyl hexyl benzoate [UVINUL^{®} A Plus, CAS: 302776-68-7], most preferably to BEMT.

It is well understood, that all preferences and definitions as given herein with regard to the ratios of D-panthenol and BEMT respectively BEMT and polysilicone-15 also apply to all uses and methods according to the present invention. The same applies for the content (amounts) of BEMT, D-panthenol and polysilicone-15. Furthermore, the amounts and ratios given for BEMT also apply to the other UVA filters, i.e. butyl methoxydibenzoyl methane and diethylamino hydroxybenzoyl hexyl benzoate.

In a particular advantageous embodiment, the uses and methods according to the present invention comprise the use of
- a weight ratio of D-panthenol to BEMT of at least 1.1; for instance from 1.1 to 5; from 1.1 to 4; from 1.1 to 3; or 1.1 to 2.5; and
- a weight ratio of polysilicone-15 to BEMT from 0.25 to 4.

Preferred topical compositions in all embodiments of the present invention are emulsions containing an oily phase and an aqueous phase such as in particular an O/W, W/O, Si/W, W/Si, O/W/O, W/O/W multiple or a pickering emulsions. The amount of the oily phase (i.e. the phase containing all oils and fats) present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the composition.

According to one preferred embodiment, the topical compositions according to the present invention are O/W emulsions comprising an oily phase dispersed in an aqueous phase in the presence of an O/W emulsifier, preferably in the presence of a cetyl phosphate, most preferably potassium cetyl phosphate.

In a further embodiment, the present invention relates to the topical composition according to the embodiments described herein for the use as sunscreen, respectively to the use of the topical composition according to the embodiments described herein as sunscreen.

In a further embodiment, the present invention relates to the use of D-panthenol for facilitating the washing out of a composition according to any one of the above described compositions out of textiles contaminated with said composition.

In a further embodiment, the present invention relates to the use of D-panthenol for reducing textile staining caused by bis-ethylhexyloxyphenol methoxyphenyl triazine or bis-ethylhexyloxyphenol methoxyphenyl triazine and polysilicone-15.

In a further embodiment, the present invention relates to a method for reducing stains on textiles by a topical composition comprising bis-ethylhexyloxyphenol methoxyphenyl triazine or bis-ethylhexyloxyphenol methoxyphenyl triazine and polysilicone-15, said method comprising the incorporation of D-panthenol into said composition.

In a further embodiment, the present invention relates to a method for facilitating the washability of topical compositions containing bis-ethylhexyloxyphenol methoxyphenyl triazine or bis-ethylhexyloxyphenol methoxyphenyl triazine and polysilicone-15 out of textiles, said method comprising the addition of D-Panthenol to the composition.

Besides the bis-ethylhexyloxyphenol methoxyphenyl triazine (BEMT) and polysilicone-15, further UV filters may be present in the topical composition according to the present invention. These UV filters are all commercially available UV-filter substances such as in particular (INCI names) methylene bis-benzotriazolyl tetramethylbutylphenol, tris-biphenyl triazine, phenylbenzimidazol sulfonic acid, 3-benzylidene camphor, octocrylene, ethylhexyl methoxycinnamate, ethylhexyl salicylate, homosalate, ethylhexyl triazone, zinc oxide, diethylhexyl butamido triazone, benzophenon-3, titanium dioxide, butyl methoxydibenzoyl methane, disodium phenyl dibenzimidazole tetrasulfonate and diethylamino hydroxybenzoyl hexyl benzoate without being limited thereto.

As the topical compositions according to the invention are intended for topical application, they comprise a physiologically acceptable medium, that is to say a medium compatible with keratinous substances, such as the skin, mucous membranes, and keratinous fibres. In particular the physiologically acceptable medium is a cosmetically acceptable carrier.

The term cosmetically acceptable carrier refers to all carriers and/or excipients and/ or diluents conventionally used in cosmetic compositions.

Preferred topical compositions according to the invention are skin care preparations, decorative preparations, and functional preparations.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment, the topical compositions according to the invention are light-protective preparations (sun care products), such as sun protection milks, sun protection lotions, sun protection creams, sun protection oils, sun blocks or day care creams with a SPF (sun protection factor). Of particular interest are sun protection creams, sun protection lotions, sun protection milks and sun protection preparations.

The topical compositions according to the present invention may be in the form of a suspension or dispersion in solvents or fatty substances, or alternatively in the form of an emulsion or micro emulsion (in particular of oil-in-water (O/W-) or water-in-oil (W/O-)type, silicone-in-water (Si/W-) or water-in-silicone (W/Si-)type, PIT-emulsion, multiple emulsion (e.g. oil-in-water-in oil (O/W/O-) or water-in-oil-in-water (W/O/W-)type), pickering emulsion, hydrogel, alcoholic gel, lipogel, one- or multiphase solution or vesicular dispersion or other usual forms, which can also be applied by pens, as masks or as sprays.

The topical compositions according to the present invention are advantageously in the form of an oil-in-water (O/W) emulsion comprising an oily phase dispersed in an aqueous phase in the presence of an O/W emulsifier, as this leads to particularly advantageous results in view of the washability. The preparation of such O/W emulsions is well known to a person skilled in the art and illustrated in the examples.

In one advantageous embodiment, the compositions in addition contain a phosphate ester emulsifier. Among the preferred phosphate ester emulsifier are C₈₋₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate. A particular phosphate ester emulsifier according to the invention is potassium cetyl phosphate e.g. commercially available as Amphisol^{®} K at DSM Nutritional Products Ltd Kaiseraugst.

If the topical composition according to the invention is an O/W emulsion, then it preferably contains at least one O/W- or Si/W-emulsifier selected from the list of PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate, PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable are commercially available polymeric emulsifiers such as hydrophobically modified polyacrylic acid such as Acrylates/C10-30 Alkyl Acrylate Crosspolymers which are commercially available under the tradename Pemulen^{®} TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable are polyalkylenglycolether such as Brij 72 (Polyoxyethylen(2)stearylether) or Brij 721 (Polyoxyethylene (21) Stearyl Ether e.g. available at Croda.

The at least one O/W respectively Si/W emulsifier is preferably used in an amount of 0.5 to 10 wt.-% such as in particular in the range from 0.5 to 5 wt.-% such as most in particular in the range from 0.5 to 4 wt.-% based on the total weight of the composition.

Suitable W/O- or W/Si-emulsifiers are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The topical compositions according to the present invention furthermore advantageously contain at least one co-surfactant such as e.g. selected from the group of mono- and diglycerides and/ or fatty alcohols. The co-surfactant is generally used in an amount selected in the range from 0.1 to 10 wt.-%, such as in particular in the range from 0.5 to 6 wt.-%, such as most in particular in the range from 1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof

The compositions in form of O/W emulsions according to the invention can be provided, for example, in all the formulation forms for O/W emulsions, for example in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection and the like).

According to an advantageous embodiment of the invention the compositions constitute cosmetic composition and are intended for topical application to the skin.

Finally, a subject-matter of the invention is a method for the cosmetic treatment of keratinous substances such as in particular the skin, wherein a composition as defined above is applied to the said keratinous substances such as in particular to the skin. The method is in particular suitable to protect the skin against the adverse effects of UV-radiation such as in particular sun-burn and/ or photoageing.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions. If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

The topical cosmetic compositions of the invention can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic compositions. Such cosmetic ingredients commonly used in the skin care industry, which are suitable for use in the compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions according to the invention in general have a pH in the range from 3 to 10, preferably a pH in the range from 4 to 8 and most preferably a pH in the range from 4 to 7. The pH can easily be adjusted as desired with suitable acids such as e.g. citric acid or bases such as NaOH according to standard methods in the art.

The topical compositions according to the invention may further contain one or more emollients which soothe and soften the skin. As an example, the emollient may be dicaprylyl carbonate or C₁₂₋₁₅alkyl benzoate. Further emollients are silicone (dimethicone, cyclomethicone), vegetable oils (grape seed, sesame seed, jojoba, etc.), butters (cocoa butter, shea butter), alcohols (stearyl alcohol, cetyl alcohol), and petrolatum derivatives (petroleum jelly, mineral oil).

The cosmetic compositions according to the present invention advantageously comprise preservatives or preservative booster. Preferably, the additional preservatives respectively preservative booster is selected from the group consisting of phenoxyethanol, ethylhexylglycerin, glyceryl caprylate, caprylyl glycol, 1,2-hexanediol, propanediol, propylene glycol as well as mixtures thereof. When present, the preservative respectively preservative booster is preferably used in an amount of 0.01 to 2 wt.-%, more preferably in an amount of 0.05 to 1.5 wt.-%, most preferably in an amount of 0.1 to 1.0 wt.-%, based on the total weight of the composition. It is particularly preferred, that the cosmetic compositions according to the invention does not contain any further/ other preservatives such as e.g. parabens and/ or methylisothiazolidine.

The following examples are provided to further illustrate the compositions and effects of the present invention. These examples are illustrative only and are not intended to limit the scope of the invention in any way.

### Experimental Part

Cream formulations as outlined in table 1 have been prepared. Afterwards, cream has been applied on a cotton fabric with the following procedure: The textile (A5 size cut piece of a cotton textile) is fixed on top of a plastic bottle with an elastic strap. Bottle with fixed textile is placed on a balance. 0.36g (+/-0.1g) of sunscreen formulation is weighed on the fabric and homogenously distributed on an area with 5 cm diameter. Then the fabric has been dried for 15 minutes followed by measuring the L,a,b value of the spot.

Afterwards, the fabric has been washed for 1h in a beaker with 300ml water and 1g detergent at 40°C under stirring, followed by rinsing the fabric in 300ml fresh water for 15 minutes. After the fabric has been dried for 15 minutes the L,a,b value of the spot has been measured again. For each trial 3 separate fabrics have been used and accordingly each result is the average of 3 fabrics.

Explanation of b value: the higher the b value, the more yellow is the fabric; the more negative the number is, the more white/bluish is the fabric.

**Table 1: Formulations**

| Ingredient | Reference | Invention |
|---|---|---|
| | Wt.-% | |
| Potassium Cetyl Phosphate | 1.5 | 1.5 |
| Stearyl Alcohol | 3.0 | 3.0 |
| Tridecyl Salicylate | 11.0 | 11.0 |
| C12-15 Alkyl Benzoate | 11.0 | 11.0 |
| Bis-Ethylhexyloxyphenol Methoxyphenyl Triazine (BEMT) | 2.5 | 2.5 |
| Polysilicone-15 | 2.5 | 2.5 |
| Aqua | q.s. | q.s. |
| Glycerin | 3.0 | 3.0 |
| Xanthan Gum | 0.3 | 0.3 |
| Phenoxyethanol, Ethylhexylglycerin | 1.0 | 1.0 |
| Panthenol | / | 5.0 |

**Table 2: Results of the washability test**

| | Reference | Invention |
|---|---|---|
| b value before washing | 1.15 | 1.44 |
| b value after washing | -0.82 | -1.38 |
| Reduction of yellow staining after washing vs. reference | - | -43% |

As can be retrieved from table 2, D-Panthenol significantly reduced the staining of a sunscreen comprising BEMT and polysilicone-15 after washing.

## Claims

1. A topical composition comprising bis-ethylhexyloxyphenol methoxyphenyl triazine, polysilicone-15 and D-panthenol, wherein the weight ratio of D-panthenol to bis-ethylhexyloxyphenol methoxyphenyl triazine is at least 1.25.

2. The topical composition according to claim 1, wherein the weight ratio is at least 1.5, preferably at least 1.75.

3. The topical composition according to claim 1 or 2, wherein the composition comprises bis-ethylhexyloxyphenol methoxyphenyl triazine in an amount selected in the range from 0.4 to 10 wt.-%, preferably from 0.8 to 6 wt.-%, more preferably from 1 to 3 wt.-%, based on the total weight of the composition.

4. The topical composition according to any one of claims 1 to 3, wherein the composition comprises D-panthenol in an amount selected in the range from 0.45 to 20 wt.-%, preferably from 0.5 to 8 wt.-%, more preferably from 0.5 to 6 wt.-%, most preferably from 0.5 to 5 wt.-%, based on the total weight of the composition.

5. The topical composition according to any one of claims 1 to 4, wherein the composition comprises polysilicone-15 in an amount selected in the range from 0.4 to 10 wt.-%, based on the total weight of the composition.

6. The topical composition according to any one of claims 1 to 5, wherein the weight ratio of polysilicone-15 to bis-ethylhexyloxyphenol methoxyphenyl triazine is selected in the range of range of 0.25 to 4.

7. The topical composition according to any one of claims 1 to 6, wherein the composition is a cosmetic or pharmaceutical composition.

8. The topical composition according to any one of claims 1 to 7, wherein the composition is a sun care product.

9. The topical compositions according to anyone of claims 1 to 8, wherein the composition is an O/W emulsion comprising an oily phase dispersed in an aqueous phase in the presence of an O/W emulsifier, preferably in the presence of a cetyl phosphate, most preferably potassium cetyl phosphate.

10. Use of D-panthenol for facilitating the washing of a composition according to any one of claims 1 to 9 out of textiles contaminated with said composition.

11. Use of D-panthenol for reducing textile staining caused by bis-ethylhexyloxyphenol methoxyphenyl triazine.

12. Method for reducing stains on textiles by a topical composition comprising bis-ethylhexyloxyphenol methoxyphenyl triazine or bis-ethylhexyloxyphenol methoxyphenyl triazine and polysilicone-15, said method comprising the incorporation of D-panthenol into said composition.

13. Method for facilitating the washability of topical compositions containing bis-ethylhexyloxyphenol methoxyphenyl triazine out of textiles, said method comprising the addition of D-Panthenol to the composition.

14. The use or the method according to anyone of claims 10 to 13, wherein the weight ratio of D-panthenol to bis-ethylhexyloxyphenol methoxyphenyl triazine is at least 1, preferably at least 1.1, more preferably at least 1.25, most preferably at least 1.75.

15. The use or the method according to anyone of claims 10 to 13, wherein the weight ratio of D-panthenol to bis-ethylhexyloxyphenol methoxyphenyl triazine is selected in the range from 1.1 to 5, preferably in the range from 1.1 to 4; from 1.1 to 3; most preferably in the range from 1.1 to 2.5.

## Patentansprüche

1. Topische Zusammensetzung, umfassend Bis-ethylhexyloxyphenolmethoxyphenyltriazin, Polysilicone-15 und D-Panthenol, wobei das Gewichtsverhältnis von D-Panthenol zu Bis-ethylhexyloxyphenolmethoxyphenyltriazin mindestens 1,25 beträgt.

2. Topische Zusammensetzung nach Anspruch 1, wobei das Gewichtsverhältnis mindestens 1,5, vorzugsweise mindestens 1,75, beträgt.

3. Topische Zusammensetzung nach Anspruch 1 oder 2, wobei die Zusammensetzung Bis-ethylhexyloxyphenolmethoxyphenyltriazin in einer Menge, die im Bereich von 0,4 bis 10 Gew.-%, vorzugsweise von 0,8 bis 6 Gew.-%, weiter bevorzugt von 1 bis 3 Gew.-%, bezogen auf das Gesamtgewicht der Zusammensetzung, ausgewählt ist, umfasst.

4. Topische Zusammensetzung nach einem der Ansprüche 1 bis 3, wobei die Zusammensetzung D-Panthenol in einer Menge, die im Bereich von 0,45 bis 20 Gew.-%, vorzugsweise von 0,5 bis 8 Gew.-%, weiter bevorzugt von 0,5 bis 6 Gew.-%, ganz besonders bevorzugt von 0,5 bis 5 Gew.-%, bezogen auf das Gesamtgewicht der Zusammensetzung, ausgewählt ist, umfasst.

5. Topische Zusammensetzung nach einem der Ansprüche 1 bis 4, wobei die Zusammensetzung Polysilicone-15 in einer Menge, die im Bereich von 0,4 bis 10 Gew.-%, bezogen auf das Gesamtgewicht der Zusammensetzung, ausgewählt ist, umfasst.

6. Topische Zusammensetzung nach einem der Ansprüche 1 bis 5, wobei das Gewichtsfeld des von Polysilicone-15 zu Bis-ethylhexyloxyphenolmethoxyphenyltriazin im Bereich von 0,25 bis 4 ausgewählt ist.

7. Topische Zusammensetzung nach einem der Ansprüche 1 bis 6, wobei es sich bei der Zusammensetzung um eine kosmetische oder pharmazeutische Zusammensetzung handelt.

8. Topische Zusammensetzung nach einem der Ansprüche 1 bis 7, wobei es sich bei der Zusammensetzung um ein Sonnenpflegeprodukt handelt.

9. Topische Zusammensetzung nach einem der Ansprüche 1 bis 8, wobei sich bei Zusammensetzung um eine O/W-Emulsion handelt, die eine in Gegenwart eines O/W-Emulgators, vorzugsweise in Gegenwart eines Cetylphosphats, ganz besonders bevorzugt Kaliumcetylphosphat, in einer wässrigen Phase dispergierte ölige Phase umfasst.

10. Verwendung von D-Panthenol zur Erleichterung des Auswaschens einer Zusammensetzung nach einem der Ansprüche 1 bis 9 aus Textilien, die mit der Zusammensetzung verschmutzt sind.

11. Verwendung von D-Panthenol zur Verringerung der durch Bis-ethylhexyloxyphenolmethoxyphenyltriazin verursachten Textilverfleckung.

12. Verfahren zur Verringerung von Flecken auf Textilien durch eine topische Zusammensetzung, die Bis-ethylhexyloxyphenolmethoxyphenyltriazin oder Bis-ethylhexyloxyphenolmethoxyphenyltriazin und Polysilicone-15 umfasst, wobei das Verfahren die Einarbeitung von D-Panthenol in die Zusammensetzung umfasst.

13. Verfahren zur Erleichterung des Auswaschens von topischen Zusammensetzungen, die Bis-ethylhexyloxyphenolmethoxyphenyltriazin enthalten, aus Textilien, wobei das Verfahren die Zugabe von D-Panthenol zu der Zusammensetzung umfasst.

14. Verwendung oder Verfahren nach einem der Ansprüche 10 bis 13, wobei das Gewichtsverhältnis von D-Panthenol zu Bis-ethylhexyloxyphenolmethoxyphenyltriazin mindestens 1, vorzugsweise mindestens 1,1, weiter bevorzugt mindestens 1,25, ganz besonders bevorzugt mindestens 1,75, beträgt.

15. Verwendung oder Verfahren nach einem der Ansprüche 10 bis 13, wobei das Gewichtsverhältnis von D-Panthenol zu Bis-ethylhexyloxyphenolmethoxyphenyltriazin im Bereich von 1,1 bis 5, vorzugsweise im Bereich von 1,1 bis 4, von 1,1 bis 3, ganz besonders bevorzugt im Bereich von 1,1 bis 2,5, ausgewählt ist.

## Revendications

1. Composition topique comprenant de la bis-éthylhexyloxyphénolméthoxyphényltriazine, de la polysilicone-15 et du D-panthénol, le rapport en poids de D-panthénol sur bis-éthylhexyloxyphénolméthoxyphényltriazine étant d'au moins 1,25.

2. Composition topique selon la revendication 1, le rapport en poids étant d'au moins 1,5, préférablement d'au moins 1,75.

3. Composition topique selon la revendication 1 ou 2, la composition comprenant de la bis-éthylhexyloxyphénolméthoxyphényltriazine en une quantité choisie dans la plage de 0,4 à 10 % en poids, préférablement de 0,8 à 6 % en poids, plus préférablement de 1 à 3 % en poids, sur la base du poids total de la composition.

4. Composition topique selon l'une quelconque des revendications 1 à 3, la composition comprenant du D-panthénol en une quantité choisie dans la plage de 0,45 à 20 % en poids, préférablement de 0,5 à 8 % en poids, plus préférablement de 0,5 à 6 % en poids, le plus préférablement de 0,5 à 5 % en poids, sur la base du poids total de la composition.

5. Composition topique selon l'une quelconque des revendications 1 à 4, la composition comprenant de la polysilicone-15 en une quantité choisie dans la plage de 0,4 à 10 % en poids, sur la base du poids total de la composition.

6. Composition topique selon l'une quelconque des revendications 1 à 5, le rapport en poids de polysilicone-15 sur bis-éthylhexyloxyphénolméthoxyphényltriazine étant choisi dans la plage de la plage de 0,25 à 4.

7. Composition topique selon l'une quelconque des revendications 1 à 6, la composition étant une composition cosmétique ou pharmaceutique.

8. Composition topique selon l'une quelconque des revendications 1 à 7, la composition étant un produit de soin solaire.

9. Compositions topiques selon l'une quelconque des revendications 1 à 8, la composition étant une émulsion O/W comprenant une phase huileuse dispersée dans une phase aqueuse en la présence d'un émulsifiant O/W, préférablement en la présence d'un cétylphosphate, le plus préférablement le cétylphosphate de potassium.

10. Utilisation de D-panthénol pour la facilitation du lavage d'une composition selon l'une quelconque des revendications 1 à 9 hors de textiles contaminés avec ladite composition.

11. Utilisation de D-panthénol pour la réduction de l'entachage de textile causé par la bis-éthylhexyloxyphénolméthoxyphényltriazine.

12. Procédé pour la réduction de tâches sur des textiles par une composition topique comprenant de la bis-éthylhexyloxyphénolméthoxyphényltriazine ou de la bis-éthylhexyloxyphénolméthoxyphényltriazine et de la polysilicone-15, ledit procédé comprenant l'incorporation de D-panthénol dans ladite composition.

13. Procédé pour la facilitation de la lessivabilité de compositions topiques contenant de la bis-éthylhexyloxyphénolméthoxyphényltriazine hors de textiles, ledit procédé comprenant l'ajout de D-panthénol à la composition.

14. Utilisation ou procédé selon l'une quelconque des revendications 10 à 13, le rapport en poids de D-panthénol sur bis-éthylhexyloxyphénolméthoxyphényltriazine étant d'au moins 1, préférablement d'au moins 1,1, plus préférablement d'au moins 1,25, le plus préférablement d'au moins 1,75.

15. Utilisation ou procédé selon l'une quelconque des revendications 10 à 13, le rapport en poids de D-panthénol sur bis-éthylhexyloxyphénolméthoxyphényltriazine étant choisi dans la plage de 1,1 à 5, préférablement dans la plage de 1,1 à 4 ; de 1,1 à 3 ; le plus préférablement dans la plage de 1,1 à 2,5.
